# DEMANDE DE BREVET EUROPEEN

(11) **EP 1 195 619 A2**
(43) Date de publication de la demande: **10.04.2002**
(21) Numéro de dépôt: 02000824.9
(22) Date de dépôt: 16.06.1996
(51) Int. Cl.: G01T 1/29

(54) **Dispositif d'analyse non invasif par radio-imagerie notamment pour l'examen in vivo de petits animaux, et procédé de mise en oeuvre**

(30) Priorité: 20.06.1995 FR 9507345
(62) Demande divisionnaire de: 96922950.9
(71) Demandeur: CENTRE NATIONAL DE LA RECHERCHE SCIENTIFIQUE (CNRS), 75016 Paris (FR)
(72) Inventeur: Mastrippolito, Roland, 78180 Montigny Le Bretonneux (FR); Ploux, Lydie, 91310 Linas (FR); Charon, Yves Pierre, 91190 Gif Sur Yvette (FR); Pinot, Laurent, 91510 Lardy (FR); Valentin, Luc, 91440 Bures Sur Yvette (FR); Valda Ochoa, Alejandro Anibal, 91400 Orsay (FR); Siebert, Rainer, 78560 Port Marly (FR); Laniece, Philippe, 75006 Paris (FR); Tricoire, Hervé, 91120 Palaiseau (FR)
(74) Mandataire: Le Forestier, Eric

(57) **Abrégé**

La présente invention concerne un dispositif d'analyse non invasif, caractérisé par le fait qu'il comprend une pluralité de détecteurs (110) associés à des structures collimatrices (120) possédant un foyer source commun (O) et des moyens de traitement (300) assurant une fonction logique combinatoire de type "ET" de la sortie des détecteurs (110) pour détecter deux rayonnements émis en coïncidence et au moins peu corrélés angulairement.

## Description

La présente invention concerne le domaine des dispositifs d'analyse non invasifs par radio-imagerie.

On a déjà proposé de nombreux dispositifs d'analyse utilisant des marqueurs radioactifs (compteurs Geiger couplés à un collimateur, gammas caméras ...).

En exploitant la propriété intrinsèque du marquage radioactif qui permet d'obtenir des informations quantitatives sur la distribution du traceur, les techniques de radioimagerie constituent un important outil tant dans le domaine clinique que dans le domaine de la recherche fondamentale.

De nos jours, les dispositifs les plus utilisés dans ce domaine appartiennent à la tomographie par émission assistée par ordinateur.

La tomographie assistée par ordinateur s'est développée selon deux modalités différentes : l'approche SPECT (Single Photon Emission Computed Tomography) qui utilise des radioisotopes émettant un photon unique par désintégration, tel que le ^{99m}Tc et le système PET (Positron Emission Tomography) qui exploite des radioisotopes dans lesquels deux rayonnements γ, sont émis simultanément lors de l'annihilation, dans le tissu, du positron issu de la désintégration, par exemple de ¹⁸F.

La plupart des systèmes SPECT est basée sur l'utilisation d'une ou plusieurs γ caméras que l'on fait tourner autour de l'objet à analyser. Une gamma-caméra typique est constituée par un collimateur multicanaux, un cristal scintillateur de grande surface, un guide de lumière pour le couplage optique entre le cristal et un ensemble de tubes photomultiplicateurs et une électronique analogique pour l'analyse de l'amplitude du signal et le codage de position. Tout le dispositif est contenu dans un blindage en plomb afin de minimiser le bruit de fond produit par des sources situées en dehors du champ de vision de la caméra. Le principe de fonctionnement de la gamma caméra est le suivant : un photon, issu d'une désintégration dans la source et qui passe le collimateur, peut interagir avec le scintillateur, provoquant une scintillation locale et isotrope. Les tubes photomultiplicateurs situés au-dessus reçoivent chacun un flux lumineux dépendant de leur distance à la source lumineuse. Il est possible alors, à partir des signaux électriques fournis par chaque photomultiplicateur, de reconstruire la position de la scintillation par barycentrage et de l'enregistrer et/ou de l'envoyer à un dispositif de visualisation.

La tomographie d'émission de positrons (PET) est une autre méthode permettant d'atteindre in vivo et non invasivement la mesure régionale de paramètres physiologiques et métaboliques. Les radioéléments émetteurs de positrons sont des isotopes ayant un excès de protons par rapport à leurs nombre de neutrons. Quand le positron est presque au repos, une rencontre avec un électron donne lieu à une réaction d'annihilation qui produit l'émission simultanée de deux photons γ partant dans des directions presque opposées. Les systèmes PET comprennent ainsi un réseau de détecteurs en couronne aptes à assurer la détection en coïncidence de deux photons, indicatrice de l'émission du positron. L'endroit de l'annihilation est alors situé quelque part dans le volume défini entre les deux détecteurs concernés.

Le document US-A-4 288 697 décrit un collimateur formé par un empilement de plaques munies de perforations répondant à une progression homothétique et réalisées par usinage chimique.

Le document IEEE Transactions On Nuclear Science, Vol 41, no. 4, décrit une structure PET classique, sans structure collimatrice focalisante.

Le document EP-A-0 289 737 décrit un scanner à point focal classique.

L'imagerie dc radiopharmaceutiques constitue un outil important dans le diagnostic, la caractérisation et le traitement des maladies et des désordres fonctionnels. Mais avant l'utilisation chez l'homme de nouveaux agents pharmacologiques, il est généralement nécessaire de les caractériser dans des modèles animaux afin de déterminer ses effets biochimiques, métaboliques et physiologiques.

Bien entendu, cette caractérisation suppose de disposer de techniques d'imagerie haute résolution afin d'évaluer, cx vivo ou in vivo, les concentrations spatiales du traceur injecté.

A l'heure actuelle, la résolution spatiale des tomographes conventionnels est de 5 à 7 mm pour les systèmes PET et de 8 à 12 mm pour les systèmes SPECT. Ces valeurs s'avèrent insuffisantes pour procéder à des études chez les petits animaux, par exemple pour étudier chez le rat, des tumeurs dont la taille typique est de quelques mm, ou la distribution de neurorécepteurs. Il est en effet nécessaire qu'un tomographe dédié à l'imagerie des petits animaux puisse fournir des résolutions spatiales d'au moins ⁻2mm.

Depuis 1990, plusieurs approches, basées sur des systèmes PET et SPECT, ont été menées pour tenter d'atteindre les performances souhaitées.

Cependant ces tentatives d'amélioration n'ont pas jusqu'ici donné satisfaction, sauf à détériorer l'efficacité de détection.

Les limitations en résolution des tomographes actuels ne permettent donc pas d'étendre les études in vivo à des modèles sur des petits animaux, pour lesquels l'expérimentation pourrait être menée de façon plus précise.

La présente invention a pour but d'améliorer cette situation.

Ce but est atteint selon la présente invention grâce à un dispositif d'analyse comprenant une pluralité de détecteurs associés à des structures collimatrices possédant un foyer source commun et des moyens de traitement des signaux provenant de la sortie des détecteurs, caractérisé par le fait que les moyens de traitement assurent une fonction logique combinatoire de type "ET" de la sortie des détecteurs pour détecter deux rayonnements émis en coïncidence et peu corrélés angulairement.

Selon une autre caractéristique de l'invention, il est prévu un collimateur multicanaux formé par empilement de plaques possédant des perforations, l'épaisseur des plaques est inférieure au diamètre des perforations dans la face interne d'entrée du collimateur et l'épaisseur de la toile entre les perforations est supérieure à l'épaisseur des plaques.

Selon une caractéristique avantageuse de l'invention, les perforations des plaques sont réalisées par usinage chimique.

La présente invention concerne également un procédé d'analyse qui comprend les étapes consistant à :
- injecter, dans un corps à analyser, un marqueur apte à générer deux rayonnements émis en coïncidence et au moins peu corrélés angulairement et
- détecter ces rayonnements grâce à un dispositif du type précité.

D'autres caractéristiques, buts et avantages de la présente invention apparaîtront à la lecture de la description détaillée qui va suivre, et en regard des dessins annexés donnés à titre d'exemple non limitatif et sur lesquels :
- la figure 1 représente une vue générale schématique d'un dispositif d'analyse conforme à la présente invention,
- la figure 2 représente une vue partiellement en coupe de la partie de détection de ce dispositif,
- la figure 3 représente schématiquement un empilement de plaques formant collimateur,
- la figure 4 représente une structure support des détecteurs,
- la figure 5 représente un schéma partiel du traitement d'un circuit d'acquisition conforme à une première variante de l'invention,
- la figure 6 représente schématiquement une conversion énergie-temps des signaux détectés, conforme à une seconde variante de l'invention,
- la figure 7 représente un schéma de circuit à cet effet
- la figure 8 représente des chronogrammes des signaux de ce circuit, et
- la figure 9 représente la structure générale d'un circuit d'acquisition.

Le dispositif d'analyse conforme à la présente invention comprend essentiellement :
- un ensemble de détecteurs 100,
- des moyens 200 adaptés pour supporter un corps à analyser C et permettre un déplacement relatif contrôlé entre celui-ci et l'ensemble de détecteurs 100, et
- des moyens de traitement 300.

L'ensemble des détecteurs 100 comprend plusieurs détecteurs 110 focalisés sur un foyer source commun O. Les détecteurs 110 sont portés par une structure support 150.

De préférence, les détecteurs 110 couvrent un angle solide au moins égal à 2π stéradians autour du foyer O.

Selon le mode de réalisation particulier et non limitatif représenté sur les figures annexées, il est prévu quinze détecteurs 110 disposés selon quinze faces adjacentes d'un icosaèdre.

Chaque détecteur 110 comprend de préférence :
- un collimateur 120,
- un scintillateur 130,
- un guide optique 135, et
- un photomultiplicateur 140.

Un tel détecteur 110 constitue un compteur de radiation γ et/ou X.

Les collimateurs 120 servent à sélectionner la direction des photons détectés. Ils sont formés de structures collimatrices focalisantes à grand angle solide.

La collimation focalisée permet de détecter de façon préférentielle la radiation provenant d'une petite région de l'espace autour du point focal O. Une telle structure collimatrice peut être formée d'une calotte sphérique, à secteurs ou d'un polyèdre constitué de pièces planes, percées de trous rayonnants coniques et construites avec un matériel de pouvoir d'absorption photoélectrique élevé.

Les trous rayonnants coniques ont de préférence au moins sensiblement les mêmes rayons d'entrée. Ils ont de préférence également au moins sensiblement les mêmes rayons de sortie, ont un même sommet et sont juxtaposés selon un réseau régulier à symétrie axiale. Les divers canaux sont séparés par des cloisons dont l'épaisseur est adaptée à l'énergie des rayonnements émis par la source de façon à pouvoir absorber une fraction importante des photons présentant une trajectoire oblique par rapport à l'axe des canaux. De cette façon, seuls les photons émis au point focal auront une chance importante de parvenir jusqu'aux scintillateurs 130.

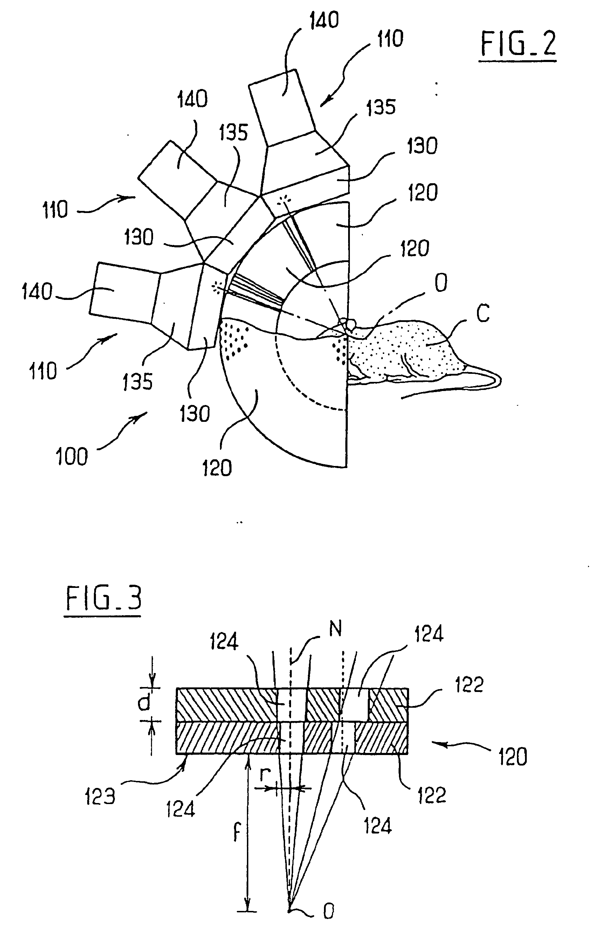

Dans le cadre de la présente invention, comme schématisé sur la figure 3, les collimateurs 120 sont de préférence réalisés par empilement de plaques perforées 122 possédant des perforations homothétiques 124.

Les plaques 122 sont avantageusement en tungstène.

Le tungstène est choisi d'une part, pour son haut pouvoir d'absorption : il présente un coefficient d'absorption supérieur de 30% à 40% à celui du plomb dans l'intervalle d'énergie 10-500 KeV. D'autre part, ses propriétés mécaniques assurent la rigidité du système et la précision de la forme des trous 124.

Par ailleurs, dans le cadre de l'invention les perforations homothétiques 124 réalisées dans les plaques de tungstène 122 sont de préférence obtenues par usinage chimique.

Après de longues études et expérimentations cette technique s'est avérée supérieure aux autres technologies de perçage connues, telles que le perçage par laser, ou l'éléctroérosion par fil.

L'usinage par attaque chimique consiste à déposer, à partir d'un masque représentant la pièce à réaliser, une résine photosensible sur toutes ses parties à préserver. La pièce est ensuite plongée dans un bain qui attaque chimiquement les zones non protégées pour former les perçages recherchés 124.

Comme cela est schématisé sur la figure 3, les trous 124 dans chaque plaque 122 sont des cylindres parallèles entre eux et orthogonaux aux faces des plaques 122. Il en résulte que l'angle solide par rapport au foyer source O sous-tendu par l'ouverture d'un trou 124 diminue au fur et à mesure que l'on s'éloigne de la normale N aux plaques 122 qui passe par le centre d'homothétie O.

Le rayon r des trous 124 dans la face d'entrée 123, l'épaisseur d des plaques 122 et la distance focale f sont déterminées pour conserver un angle solide acceptable sous-tendu par l'ouverture de chaque trou 124 par rapport à O.

L'épaisseur d des plaques 122 est de préférence inférieure au diamètre des trous 124 dans la face interne, d'entrée 123, du collimateur, par exemple égale à la moitié de ce diamètre, et l'épaisseur de la toile (septas) entre les trous 124, dans cette face interne 123 est supérieure ou égale à l'épaisseur des plaques 122, (soit par exemple une distance entre centre des trous à égale à trois fois le rayon des trous 124 sur la face interne 123).

Pour un assemblage des détecteurs 110 sur un support polyédrique 150 du type icosaèdre régulier, chaque module de collimation 120 prend la forme d'un tronc de pyramide triangulaire.

Selon un mode de réalisation particulier et non limitatif, chaque collimateur 110 est formé par empilement de 48 plaques 122 de tungstène, d'une épaisseur de 0,2mm, présentant un réseau de trous 124 définissant une distance focale f de l'ordre de 7cm, les trous 124 ayant dans la face interne d'entrée 123, du collimateur, un rayon de l'ordre de 0,2mm et un entraxe de l'ordre de 0,5mm.

Plus précisément, chaque collimateur 110 peut être formé par empilement de 24 paires différentes de plaques 122 identiques deux à deux. Dans une telle disposition, chaque plaque d'épaisseur d est constituée par la juxtaposition de deux écrans identiques d'épaisseur d/2. De cette façon, on peut aisément réaliser des trous 124 de diamètre égal à d, dont la séparation entre centres est égale à 3d/2.

Plus précisément encore selon un mode de réalisation très particulier et non limitatif, le rayon des trous 124 est de 0,205mm dans la première plaque 122 et de 0,231mm dans la dernière plaque 122, la distance entre centres des trous 124 est de 0,614 mm dans la première plaque 122 et de 0,693mm dans la dernière plaque 122, la longueur des arêtes de la première plaque 122 est de 87,2 mm et de 98,3 mm pour la dernière plaque 122, et la distance au point focal est de 71,6 mm pour la première plaque 122 et de 80,8 mm pour la dernière plaque 122.

Le scintillateur 130 est de préférence formé d'un monocristal d'iodure de sodium activé au thallium (NaI(T1)).

Pour une disposition de type icosaédrique, le cristal scintillateur a la forme d'un prisme à section triangulaire afin de couvrir toute la surface de sortie du collimateur.

Le guide de lumière 135 assure le couplage optique entre chaque cristal scintillateur 130 et le tube photomultiplicateur associé 140.

La structure 150 supportant les détecteurs 110 doit assurer le positionnement des points focaux des collimateurs 120 avec une précision suffisante, typiquement de l'ordre de 0,1 mm. Une telle structure support 150 peut faire l'objet de nombreuses variantes de réalisation.

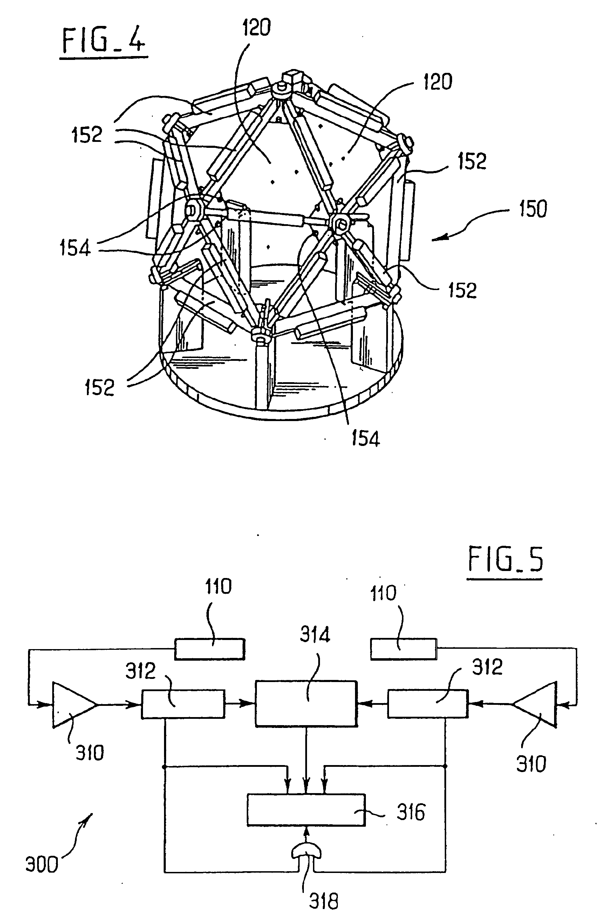

Un mode de réalisation particulier comprenant une armature constituée de barres support 152 reliées 5 à 5 par leurs extrémités et agencées selon les arêtes d'un icosaèdre est représenté sur la figure 4. Les plaques 122 des collimateurs 120 peuvent être fixées sur ces barres 152 grâce à des piges 154 engagées dans les angles des plaques 122.

Cependant en variante la structure 150 peut supporter les guides de lumière 135 des détecteurs et non pas les collimateurs 120.

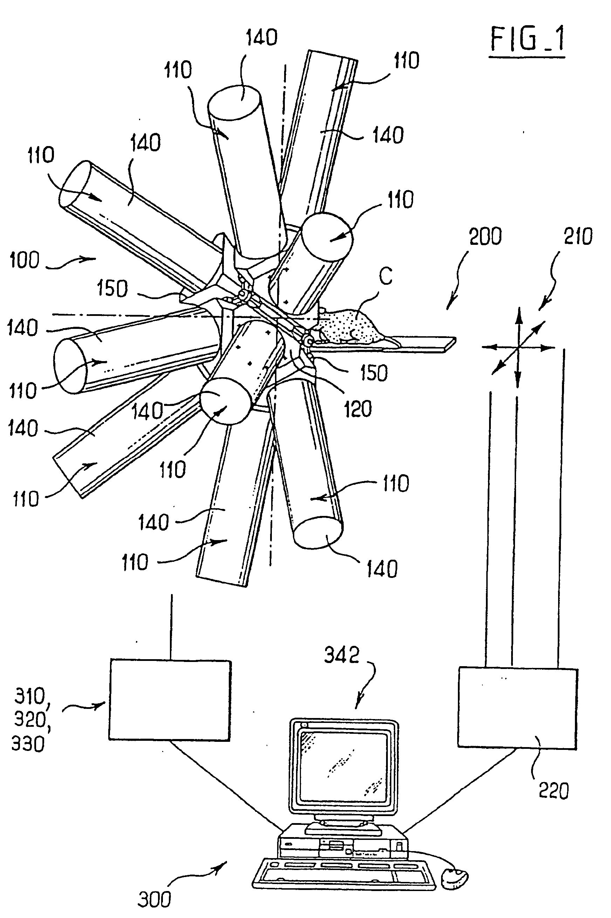

Le système 200 de support et de déplacement de l'objet analysé est adapté pour définir trois degrés de liberté de déplacement linéaire à l'objet analysé C par rapport aux capteurs 110. Ces déplacements peuvent étre obtenus à l'aide de trois axes motorisés contrôlés et repérés, schématiquement représentés en 210 sur la figure 1, mutuellement orthogonaux associés à des moyens de commande 220 assurant des déplacements des moteurs interactivement afin de positionner l'objet C et de définir une région d'analyse et un pas de déplacement afin d'effectuer automatiquement le balayage nécessaire à l'acquisition.

La mécanique 210 de déplacement de chaque axe peut être composée des unités suivantes pour chaque axe motorisé
- une platine de translation qui entraine le mouvement d'un chariot par un système vis-écrou, sur une course par exemple de 10 cm, et
- un moteur pas à pas qui permet sur chaque platine de translation des incréments contrôlés par exemple de 10µm.

L'image résultant d'une acquisition est construite à partir du nombre de photons détectés à chaque position du système de déplacement 210. Durant l'acquisition il est donc nécessaire d'éviter tout mouvement de l'objet analysé C, par rapport au système de déplacement 210, afin de ne pas introduire d'artefacts dans l'image.

Le système 200/210 doit par conséquent être équipé d'un système de positionnement spécifique de l'objet analysé.

A titre d'exemple non limitatif, pour l'imagerie cérébrale d'un rat il suffit simplement d'immobiliser la tête de celui-ci. Le dispositif de positionnement utilisé dans ce cas peut correspondre à ceux connus dans les appareils pour stéréotaxie. L'immobilisation de la tête dans ce cas, s'effectue en trois points : aux entrées des orifices auriculaires, avec deux barres de position réglable, et derrière les incisives, avec une barre sur laquelle s'appuie la mâchoire supérieure.

Les moyens de traitement 300 sont adaptés pour détecter deux rayonnements en coïncidence temporelle produits sur l'ensemble des détecteurs 110, sans éventuellement prendre en considération les coïncidences produits dans un même détecteur ni celles produites dans des détecteurs diamétralement opposés.

Ainsi, la détection d'un événement se fait avec une probabilité d'autant plus élevée qu'il provient du point focal grâce à la conjugaison d'une collimation physique, donnée par la strucutre collimatrice, et d'une collimation électronique, donnée par la détection en coïncidence des photons issus de la désintégration.

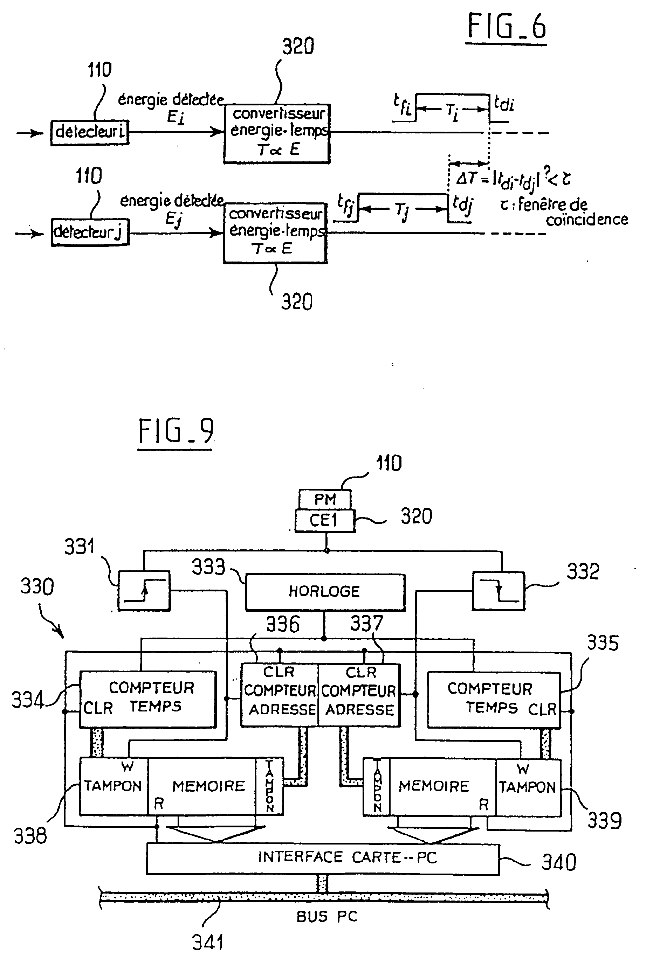

Lorsqu'il s'agit de détecter deux photons de même énergie, l'électronique 300 de traitement et d'acquisition peut être particulièrement simple, comme représenté sur la figure 5.

Chaque ligne de détection associée à un détecteur 110 comprend un amplificateur 310 et un module discriminateur 312 définissant une fenêtre autour du photopic. Les sorties des analyseurs monocanaux 312 sont utilisées pour générer le signal de coïncidence dans un convertisseur temps-amplitude 314. Trois signaux sont envoyés à une carte d'interface 316 : le signal de coïncidence issu du convertisseur 314 et les deux sorties des analyseurs monocanaux 312, ces dernières dans le but de construire une image monophotonique pour chaque détecteur 110. Un signal d'acquisition généré par combinaison logique dans une porte 318, des images d'événements simples provenant des discriminateurs 312, est également appliqué à la carte d'interface 316.

Le dispositif décrit précédemment et illustré sur la figure 5 devient cependant rapidement lourd et impraticable dès lors que l'on veut détecter en coïncidence, et parmi plus de deux détecteurs 110, deux rayonnements d'énergie différente.

Pour résoudre cette difficulté, il est proposé dans le cadre de l'invention un dispositif d'acquisition spécifique qui permet pour chaque événement détecté, d'extraire l'énergie de l'événement et l'instant auquel il s'est produit, afin de tester sa coïncidence temporelle avec un autre évènement.

Un tel dispositif assure par exemple une conversion énergie-temps et code les événements dans un signal dont la largeur est proportionnelle à leur énergie. Un tel système permet de coder aussi, de façon intrinsèque, l'instant d'arrivée de chaque événement, et permet alors des tests de coïncidence.

Le principe de fonctionnement d'un système de détection de coïncidences basé sur un convertisseur énergie-temps est le suivant. Deux événements sont détectés sur les détecteurs i et j respectivement. Chaque convertisseur énergie-temps produit un signal dont la largeur T est proportionnelle à l'énergie déposée dans le détecteur. Si les largeurs Ti et Tj correspondent aux énergies en jeu, on considère les événements comme coïncidents si la différence de débuts de signaux, t_{di}-t_{dj}, est inférieur à la largeur de la fenêtre de coïncidences τ fixée préalablement.

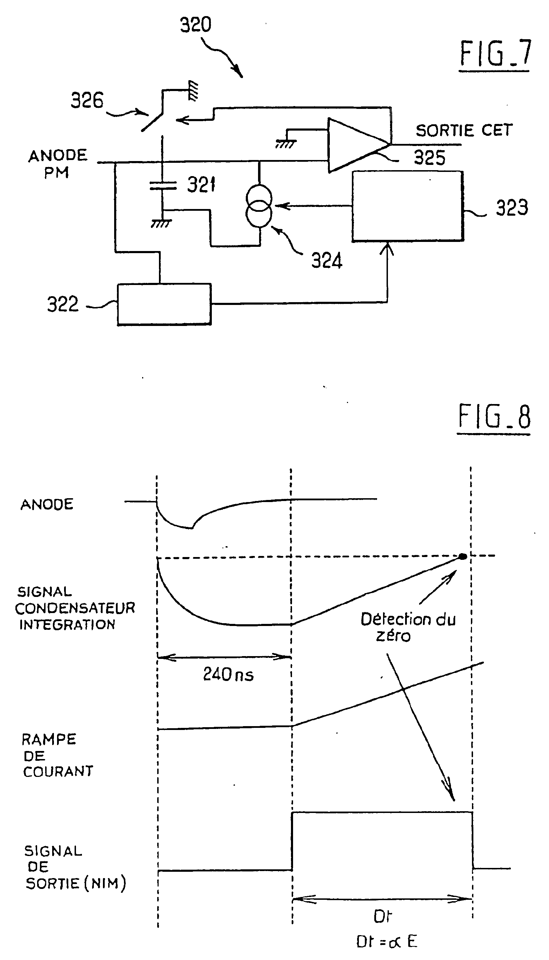

On a représenté sur la figure 7 un exemple de réalisation d'un circuit 320 conforme à la présente invention apte à assurer une telle conversion énergie/temps, et sur la figure 8 des chronogrammes des signaux pris sur différents points du circuit.

Le circuit de la figure 7 comprend :
- un condensateur 321 apte à intégrer le signal issu d'un photomultiplicateur 140,
- un détecteur 322 de signal en sortie du photomultiplicateur,
- une cellule à retard 323 initiée par le détecteur précité 322,
- une source de courant 324 montée en parallèle du condensateur 321 et qui est pilotée par la sortie de la cellule à retard 323.
- un comparateur 325 dont une entrée est à la masse et l'autre entrée est reliée au condensateur 321, la sortie de ce comparateur 325 constituant la sortie du dispositif de conversion énergie/temps, et
- un interrupteur 326 commandé par le comparateur 325 et apte à décharger le condensateur 321.

Le fonctionnement de ce circuit 320 est le suivant.

La lumière de scintillation produite par l'interaction d'un photon X ou γ dans le cristal 130 de NaI(TI), se manifeste, sur l'anode du photomultipliacteur 140, par un signal ayant une montée (en valeur absolue) très rapide suivie d'une descente presque exponentielle typiquement de l'ordre de 230 ns de temps caractéristique. Comme l'intégrale de cette réponse est proportionnelle à l'énergie déposée par la radiation dans le cristal, le but du convertisseur énergie/temps 320 est de récupérer cette intégrale pour moduler la largeur d'un signal carré. L'intégrale du signal de l'anode est récupérée sur le condensateur 321 pendant un temps prédéfini. Ce temps est obtenu à partir d'un retard appliqué au signal extrait de la dernière dynode du photomultiplicateur 140 (modules Détection signal 322 et cellule retard 323). A la fin du temps d'intégration, le générateur de courant 324, connecté en parallèle du condensateur 321, est mis en service afin de produire une décharge linéaire de celui-ci. Simultanément au démarrage du générateur de courant 324, la sortie de la bascule 325 qui donne le signal de sortie du convertisseur 320, passe à un état logique "1". Lors du passage par zéro de la tension du condensateur 321, la bascule 325 retourne à son état bas, interrompant ainsi le niveau haut du signal de sortie, le générateur de courant 324 est arrêté et le condensateur 321 est complètement déchargé en activant l'interrupteur 326.

On a représenté sur la figure 9, un exemple de réalisation d'un circuit d'acquisition 330 conforme à la présente invention.

Ce circuit 330 est adapté pour coder des énergies allant par exemple de ⁻10 keV (27 keV pour ¹²³I et ¹²⁵I) jusqu'à -300 keV (245 keV pour ¹¹¹In) à un taux de comptage maximal de ⁻10⁴ coups par seconde par détecteur.

Le système 330 est composé par exemple de :
- un ensemble de (par exemple 15) détecteurs (scintillateur 130 + photomultiplicateur 140),
- un convertisseur énergie/temps 320 couplé à chaque détecteur 110,
- deux détecteurs 331, 332 respectivement de fronts montant et descendant des signaux issus des convertisseurs 320,
- une horloge 333 pour la référence temporelle des signaux,
- deux compteurs de temps 334, 335 pilotés par l'horloge 333,
- deux compteurs d'adresse 336, 337 pilotés par les détecteurs de front 331, 332, et
- deux mémoires 338, 339 pour le stockage cyclique et temporaire des informations puis le transfert sur le bus 341 d'un ordinateur 342.

A la sortie de chaque convertisseur énergie/temps 320 on trouve un signal dont la largeur est proportionnelle à l'énergie déposée dans le détecteur 110. Cette largeur peut varier par exemple de ⁻10 ns pour 3 keV jusqu'à ⁻1µs pour 300 keV. Ceci permet aisément un taux de comptage de 10⁵ coups par seconde. Les instants des fronts de montée et de descente du signal sont calculés à l'aide des compteurs de temps 334, 335 et de l'horloge 333 et sont stockés dans les mémoires indépendantes 338, 339. Une fois terminée l'acquisition dans un voxel, les données des mémoires 338, 339 sont transférées au bus 341 du PC afin d'être traitées par un logiciel spécifique.

Le procédé d'acquisition d'informations, conforme à la présente invention, en vue d'une imagerie, comprend bien entendu l'étape initiale d'injection dans le corps à analyser C, d'un marqueur radioactif apte à émettre deux rayonnements en coïncidence, au moins peu corrélés angulairement.

Un tel marqueur peut faire l'objet de nombreuses variantes.

Il peut faire appel au moins à trois mécanismes de désintégration radioactive donnant lieu à l'émission de deux photons en coïncidence peu corrélés angulairement.

Dans le premier des mécanismes de désexcitation (désexcitation isobarique d'un noyau), la désintégration d'un noyau père produit un noyau excité qui passe à son état fondamental par l'intermédiaire d'une cascade, produisant ainsi deux gammas en coïncidence. Un exemple de radioélément se désexcitant par ce mécanisme est ¹¹¹In. Après une capture électronique, ¹¹¹In (demi-vie : 2,8 jours), passe essentiellement au niveau excité de 417 keV de ¹¹¹Cd. L'état fondamental de ¹¹¹Cd est atteint par l'émission en cascade d'un gamma de 171 keV suivi d'un autre de 245 keV ; la demi-vie du niveau de 245 keV est de 85 ns.

Les deux autres mécanismes concernent les radioéléments dont la désintégration débute par une capture électronique, laquelle sert à fournir le premier photon de la paire en coïncidence. En effet, dans la capture électronique on met à profit l'émission Xₖ, issue du réarrangement des couches électroniques profondes, pour disposer d'un premier photon. La désexcitation du noyau fils donne lieu au deuxième photon et pour cela on trouve deux possibilités. Si la désexcitation est radiative, il y aura un photon γ en coïncidence avec le photon Xₖ. Si la désexcitation se réalise au moyen d'une conversion interne, il y aura aussi un réarrangement électronique et une émission d'un photon Xₖ en coïncidence avec celui de la capture électronique. Deux isotopes de l'iode (Z=53) servent d'exemple à ces deux mécanismes de coïncidences : ¹²³I (demi-vie : 13 h) et ¹²⁵I (demi-vie : 60j). Dans les deux cas on a la radiation Xₖ émise d'environ 27 keV, la probabilité de réaliser la capture d'un électron de la couche K de 80% et le rendement de fluorescence de 86%. Dans le cas de ¹²³I, la capture électronique conduit à un écat excité de ¹²³Te qui passe au fondamental par l'émission d'un gamma de 159 keV, le taux de coïncidence Xₖ-_{γ} est d'environ 70%. Dans le cas de ¹²⁵I, l'état excité du noyau fils à une énergie de 35 keV, le passage au fondamental s'effectue soit par émission d'un gamma (7% de probabilité) soit par une conversion interne ; le taux de coïncidences Xₖ-_{γ} plus Xₖ-Xₖ est de 60%.

L'invention n'est pas limitée à l'utilisation des radioéléments précités : ¹²³I, ¹²⁵I et ¹¹¹In, mais s'étend à tout autre radioélément équivalent apte à émettre au moins deux rayonnements en coïncidence temporelle, tels que γ-γ, γ-X ou X-X, et au moins peu corrélés angulairement.

Après ou avant injection du radiotraceur, le corps à analyser C est placé dans la structure de détection collimatrice 100.

Les radiations émises par l'objet C, et traversant les collimateurs 120 sont détectées par l'ensemble des détecteurs 110 (scintillateurs 130 + photomultiplicateurs 140). Le signal issu des détecteurs 110, amplifié et traité dans les moyens 310, 320, 330, est envoyé sur un ordinateur 342 qui est chargé de son acquisition et de son stockage. L'électronique de traitement 310, 320, 330, fournit soit les signaux de coïncidence entre deux détecteurs 110 quelconques, soit un ensemble de signaux afin de pouvoir récupérer, a posteriori, les coïncidences produites.

La détection en coïncidence permet l'optimisation de la résolution spatiale et le rejet des signaux provenant de points situés en dehors du point focal.

Le déplacement par balayage du point focal des collimateurs 120 dans le volume de la région d'intérêt au cours de l'acquisition permet de construire l'image de l'objet C voxel par voxel. Ainsi, il n'est pas nécessaire d'utiliser un algorithme de reconstruction de l'image susceptible notamment d'amplifier les fluctuations statistiques sur l'image reconstruite comme dans le cas des techniques SPECT et PET, et la visualisation peut se faire selon l'invention, voxel par voxel, au fur et à mesure du déroulement de l'acquisition.

La présente invention peut donner lieu à de nombreuses applications, nécessitant la mesure de concentration d'espèces chimiques marquées radioactivement, parmi lesquelles on peut citer, de manière non limitative, l'examen in vivo de petits animaux, notamment dans le cadre de recherches cliniques, concernant par exemple la détection de lésions cardiovasculaires, la cancérologie, la détection et le suivi de tumeurs, l'étude de la distribution de neurorécepteurs, la visualisation des fonctions cérébrales (lors de maladies neurodégéneratives, comme les maladies de Parkinson ou d'Alzheimer ou encore d'Huntington ; ou lors des désordres psychiatriques, comme dans la schizophrénie), la thérapie génique ou d'une façon plus générale la neurobiologie ou la neuropharmacologie afin d'évaluer l'efficacité des thérapies basées sur l'administration d'agents neuroprotecteurs et sur l'implantation de greffes neuronales.

L'utilisation du dispositif et la mise en oeuvre du procédé précédemment décrits peuvent être conduites par toute personne autorisée sans exiger de connaissance particulière dans le domaine médical.

## Revendications

1. Collimateur pour dispositif d'analyse par radioimagerie, **caractérisé par le fait qu'**il est formé d'un empilement de plaques (122) munies de perforations (124), l'épaisseur (d) des plaques (122) est inférieure au diamètre des perforations (124) dans la face interne d'entrée (123) du collimateur et l'épaisseur de la toile entre les perforations (124) est supérieure à l'épaisseur des plaques (12).

2. Collimateur selon la revendication 1, **caractérisé par le fait que** les perforations (124) prévues dans les différentes plaques (122) présentent une progression homothétique.

3. Collimateur selon l'une des revendications 1 ou 2, **caractérisé par le fait que** les perforations (124) sont réalisées par usinage chimique.

4. Collimateur selon l'une des revendications 1 ou 3, **caractérisé par le fait que** les plaques (122) sont réalisées en tungstène.

5. Collimateur selon l'une des revendications 1 à 4, **caractérisé par le fait que** certaines au moins des plaques (122) sont formées par superposition de deux écrans perforés identiques.
